# EUROPEAN PATENT APPLICATION

(11) **EP 2 759 316 A2**
(43) Date of publication of application: **30.07.2014**
(21) Application number: 12827104.6
(22) Date of filing: 23.08.2012
(51) Int. Cl.: A61N 5/06

(54) **APPARATUS FOR IRRADIATING SKIN USING LIGHT**

(30) Priority: 02.09.2011 KR 20110088800
(71) Applicant: Ceragem Medisys Inc., Cheonan-si, Chungcheongnam-do 331-833 (KR)
(72) Inventor: LEE, Jin Woo, Cheonan-si Chungcheongnam-do 331-833 (KR); CHOI, Jae Kyu, Cheonan-si Chungcheongnam-do 331-833 (KR)
(74) Representative: Bridge, Kerry Ann
(86) International application number: PCT/KR2012/006724
(87) International publication number: WO 2013/032176

(57) **Abstract**

The present invention relates to an apparatus for irradiating skin with light for preventing a user's eyes from being exposed to medical light. An apparatus for irradiating skin with light according to the present invention comprises an input unit for a user input, a light source unit including light sources of at least two colors and configured to irradiate the skin of a user with medical light, a contact sensor provided at periphery of the light source unit, and a controller configured to control an intensity of the light radiated from the light source unit according to whether skin contact is detected by the contact sensor, wherein the controller controls the intensity of the light from the light source unit in at least two stages before and after the skin contact is detected. Therefore, the present invention has an advantage in which safety is improved because the user's eyes are not exposed to high-intensity medical light.

## Description

### [Technical Field]

The present invention relates to an apparatus for irradiating skin with light for skin treatment, and more particularly to an apparatus for irradiating skin with light capable of improving safety and usability.

### [Background Art]

Recently, an acne treatment device with a light source unit configured to emit light such as a laser or a light-emitting diode (LED) has been developed.

When acne is treated using a conventional acne treatment device with the light source unit as described above, there is a risk in that a user's eyes may be directly exposed to light emitted from the light source unit. Although the above-described light source unit emits laser light, ultraviolet light, or the like with a proper intensity in consideration of skin risk, there is a problem in that the exposure of the eyes to the light may result in blindness. In particular, dangerousness due to unprofessional or careless treatment when a patient himself/herself directly operates the treatment device for self treatment or dangerousness due to possibility of incidental manipulation of children should be considered very seriously.

In addition, the above-described problem is still present even when a device using light is used during treatment for various types of skin ailments and treatment for improvement of a skin state as well as acne treatment.

In order to solve this problem, technology for adjusting generation of light by detecting contact with skin so that it is possible to prevent light emitted from the light source unit from being directly radiated to the eyes when the skin is treated using light has been developed in the related art.

However, there are some problems in a conventional apparatus for irradiating the skin with the light so as to adjust the generation of the light by detecting the contact with the skin as described above.

First, the conventional apparatus for irradiating the skin with the light is configured so that the light is radiated from the light source unit immediately when the skin contact is detected. In general, a predetermined time is required in a process in which the user causes the conventional apparatus for irradiating the skin with the light to be completely closely in contact with his/her skin after causing the conventional apparatus to be in contact with the skin. When the conventional apparatus for irradiating the skin with the light is used, there is a risk in that the light is radiated and exposed to the user's eyes for the predetermined time. In addition, the conventional apparatus for irradiating the skin with the light irradiates the skin with high-intensity light immediately when it is in contact with the user's skin. When the user separates the conventional apparatus for irradiating the skin with the light from the skin before the conventional apparatus is completely closely in contact with the skin (for example, when the conventional apparatus is moved to an accurate portion to be treated due to wrong selection of a portion to be treated, when the conventional apparatus is separated from the skin so as to receive a phone call or makes a response to a call from someone, or the like), there is a problem in that the user is dangerous if the eyes are irradiated with high-intensity light.

Second, there is a risk in that the eyes may be exposed to light when the user momentarily separates the conventional apparatus for irradiating the skin with the light from the skin because a reaction rate of the controller to a detection signal of a contact sensor is slow. That is, the controller checks whether the contact sensor generates the detection signal indicating that the conventional apparatus is separated from the skin at intervals of several seconds. Because light irradiation is not immediately stopped even when the conventional apparatus is separated from the user's skin when the controllers checks at the intervals of several seconds, the user's eyes are directly exposed to the light.

Third, when the contact sensor is configured to surround a head portion of the conventional apparatus for irradiating the skin with the light, there is a risk in that the eyes are directly irradiated with light when a hand is in contact with the head portion and the contact sensor detects the contact. That is, the light irradiation should be achieved only when the conventional apparatus is in contact with the user's skin. Even when the conventional apparatus for irradiating the skin with the light is in contact with the user's hand or the like other than desired skin, the light irradiation starts and the light is directly exposed to the user's eyes. In addition, because only one contact sensor is disposed around the light source unit, the conventional apparatus for irradiating the skin with the light is configured to radiate light immediately when any part of the contact sensor is in contact with the skin. Because it is difficult to detect whether the light source unit is completely in contact with the user's skin through the above-described configuration, there may be a problem in that the user's eyes are exposed to light.

Because there is a possibility of a symptom such as cataract or glaucoma as well as vision problems when the user's eyes are iteratively exposed to light as described above, improvement thereof is necessary.

Fourth, because the conventional apparatus for irradiating the skin with the light has been usually developed in a portable type, there is a problem in that a button may be erroneously pressed when the conventional portable apparatus is carried and the power supply may be turned on. That is, because power of an embedded power supply unit may be unnecessarily consumed when the power supply is turned on due to erroneous pressing of the button, the apparatus may not be used at a desired time due to the power consumption, an erroneous operation of the contact sensor may be caused, and a life of the light source unit may also be shortened due to a frequent operation of the light source unit, improvement thereof is necessary.

### [Disclosure]

### [Technical Problem]

The present invention has been proposed to solve the above-described problems and an object of the present invention is to provide an apparatus for irradiating skin with light that may detect whether the apparatus is closely in contact with a user's skin when the apparatus is used and radiate medical light when the apparatus is closely in contact with the user's skin.

Another object of the present invention is to provide an apparatus for irradiating skin with light that may improve safety by preventing light from being exposed to a user's eyes because irradiation of medical light is stopped rapidly when the apparatus is separated from the skin by increasing a reaction rate according to detection of the presence/absence of skin contact.

Still another object of the present invention is to provide an apparatus for irradiating skin with light that may perform light irradiation suitable for the purpose of radiation by selectively radiating one of light sources of at least two colors or sequentially radiating the light sources of the at least two colors.

### [Technical Solution]

According to the present invention for achieving the above-described objects, there is provided an apparatus for irradiating skin with light, comprising: an input unit for a user input; a light source unit including light sources of at least two colors and configured to irradiate the skin of a user with medical light; a contact sensor provided at periphery of the light source unit; and a controller configured to control an intensity of the light radiated from the light source unit according to whether skin contact is detected by the contact sensor, wherein the controller controls the intensity of the light from the light source unit in at least two stages before and after the skin contact is detected.

The controller may drive the light source unit with a first light intensity before the skin contact is detected by the contact sensor after a voltage is applied, and the controller may drive the light source unit with a second light intensity when the skin contact is detected.

The controller may decrease the intensity of the light from the light source unit to the first light intensity when the skin contact is released while the medical light is radiated.

The controller may drive the light source unit for a set driving time if the skin contact is not released while the medical light is radiated, and the controller may decrease the intensity of the light from the light source unit to the first light intensity when the driving time has expired.

The controller may cause the light intensity to be gradually increased until the light source unit reaches the first or second light intensity.

The controller may selective or sequentially drive at least one of the light sources of the at least two colors.

The light sources of the at least two colors may include a blue light source and a red light source, the controller may drive only one of the blue light source and the red light source according to the input to the input unit, and the controller may first drive one of the blue light source and the red light source according to the input to the input unit and subsequently drive the other.

Preferably, the apparatus for irradiating the skin with the light may further include at least one of: a display unit configured to display various types of device states; a temperature sensor configured to detect a temperature so as to prevent an over-temperature of the light source unit; and a notification sound generation means configured to output a notification sound.

A plurality of contact sensors may be provided to oppose each other around the light source unit, and the controller may start to radiate the medical light only when the plurality of contact sensors all detect the skin contact.

Preferably, the apparatus for irradiating the skin with the light may further include: an optical detection unit configured to detect external light, and the controller may start to radiate the medical light when the external light is not detected by the optical detection unit.

### [Advantageous Effects]

Advantageous effects of the apparatus for irradiating the skin with the light according to the present invention are as follows.

First, there is an advantage in that the safety of the apparatus is improved by radiating high-intensity medical light when the apparatus is completely closely in contact with a user's skin according to the present invention.

Second, there is an advantage in that the safety of the apparatus is improved by preventing light from being exposed to a user's eyes because irradiation of medical light is stopped rapidly when the apparatus is separated from the skin by increasing a reaction rate according to detection of the presence/absence of skin contact according to the present invention.

Third, there is an advantage in that the apparatus may be driven to be suitable for the purpose of radiation by providing light sources of at least two colors in a light source unit and selectively or sequentially driving the light sources.

### [Description of Drawings]

FIG. 1 is a schematic diagram illustrating a configuration of an apparatus for irradiating skin with light according to the present invention.
FIGS. 2a and 2b illustrate external appearances of the apparatus for irradiating the skin with the light according to the present invention, wherein FIG. 2a is a front view and FIG. 2b is a rear view.
FIGS. 3a and 3b are graphs each illustrating a light intensity control method for medical light according to a first embodiment of the present invention, wherein FIG. 3a is a graph illustrating voltage pulse control according to an operation process and FIG. 3b is a graph illustrating a light intensity variation amount according to the operation process.
FIGS. 4a and 4b are graphs each illustrating a pulse-width modulation (PWM) method among light intensity control methods for the medical light according to a second embodiment of the present invention, wherein FIG. 4a is a graph illustrating the adjustment of a voltage pulse width according to time and FIG. 4b is a graph illustrating a light intensity variation amount according to time.
FIGS. 5a and 5b are graphs each illustrating a pulse-frequency modulation (PFM) method among the light intensity control methods for the medical light according to the second embodiment of the present invention, wherein FIG. 5a is a graph illustrating the adjustment of a voltage pulse frequency according to time and FIG. 5b is a graph illustrating a light intensity variation amount according to time.
FIGS. 6a and 6b are graphs each illustrating an application voltage adjustment method among the light intensity control methods for the medical light according to the second embodiment of the present invention, wherein FIG. 6a is a graph illustrating the adjustment of an application voltage according to time and FIG. 6b is a graph illustrating a light intensity variation amount according to time.
FIG. 7 is a flowchart illustrating a method of operating the apparatus for irradiating the skin with the light according to the first embodiment of the present invention.
FIG. 8 is a flowchart illustrating a method of operating the apparatus for irradiating the skin with the light according to the second embodiment of the present invention.

### [Modes of the Invention]

Hereinafter, embodiments of an apparatus for irradiating skin with light according to the present invention will be described in detail with reference to the drawings.

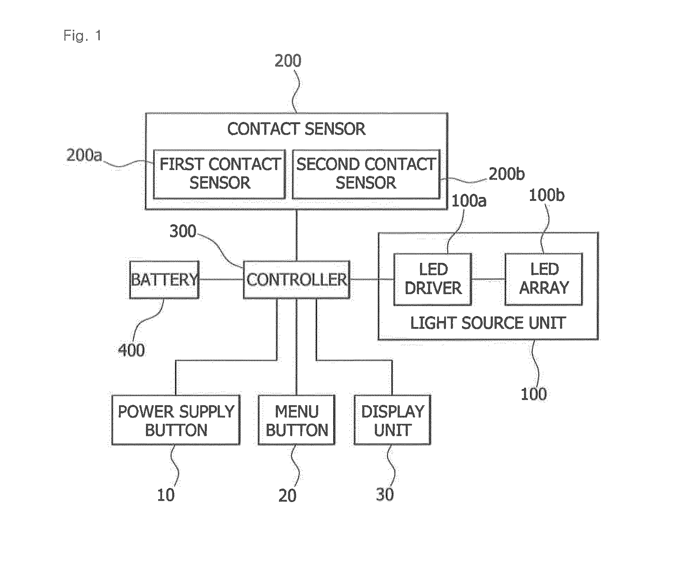

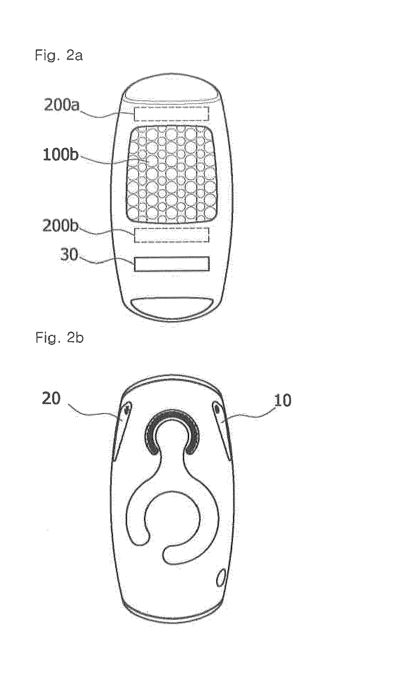

FIG. 1 is a schematic diagram illustrating a configuration of the apparatus for irradiating the skin with the light according to the present invention and FIG. 2a and 2b illustrate external appearances of the apparatus for irradiating the skin with the light according to the present invention, wherein FIG. 2a is a front view and FIG. 2b is a rear view.

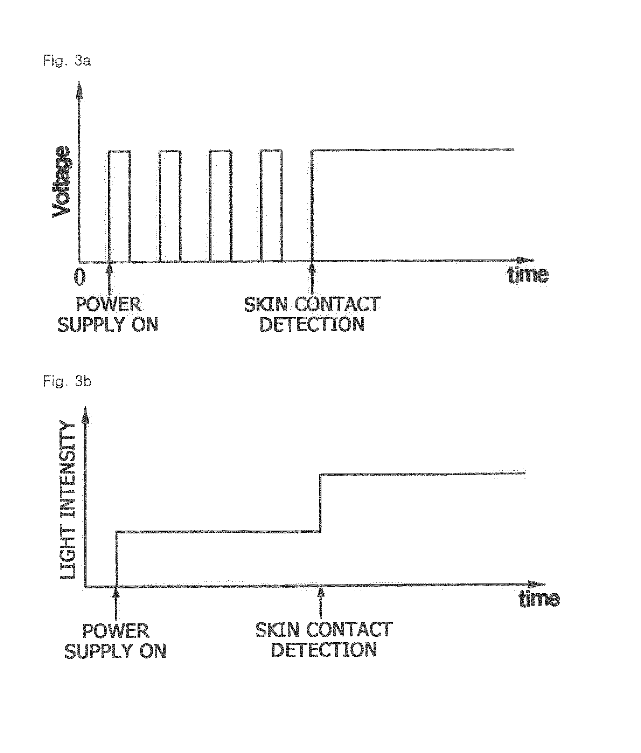

FIGS. 3a and 3b are graphs each illustrating a light intensity control method for medical light according to a first embodiment of the present invention, wherein FIG. 3a is a graph illustrating voltage pulse control according to an operation process and FIG. 3b is a graph illustrating a light intensity variation amount according to the operation process.

As illustrated in FIGS. 1 and 2, the apparatus for irradiating the skin with the light according to the present invention comprises a power supply button 10, a menu button 20, a display unit 30, a light source unit 100 [an LED driver 100a and an LED array 100b], a contact sensor 200 [a first contact sensor 200a and a second contact sensor 200b], a controller 300, a battery 400, etc.

Preferably, the apparatus for irradiating the skin with the light according to the present invention may be used for self treatment of acne and may also be used for treatment of various types of skin ailments and treatment for improvement of a skin state as well as acne treatment.

Accordingly, in the apparatus for irradiating the skin with light according to the present invention, the light source unit 100 may include light sources of at least two colors. That is, the LED array 100b may include LEDs of at least two colors. For example, first colored LEDs may be aligned and disposed and second colored LEDs may be aligned and disposed between the first colored LEDs. Preferably, the first colored LED may be a blue LED and the second colored LED may be a red LED.

It is preferable to determine a type of light source of the light source unit 100, a wavelength band of irradiation light, etc. according to the purpose of the apparatus for irradiating the skin with the light. For example, when the apparatus for irradiating the skin with the light according to the present invention is configured for acne treatment, the LED array 100b may be implemented with a plurality of blue LEDs with a wavelength of 400 nm and a plurality of red LEDs with a wavelength of 600 nm. Of course, the apparatus for irradiating the skin with the light according to the present invention may use light sources such as microwaves, high-frequency waves, ultraviolet light, visible light, infrared light, ultrasonic waves, laser light, etc. and wavelength bands thereof and may use light sources such as an LED, a Xenon light, a laser diode, etc. and light of wavelength bands thereof.

The power supply button 10 and the menu button 20 may be implemented as at least one input unit and support a partner function. For example, when the menu button 20 is pressed, it operates along with the power supply button 10 and a power supply may be turned on.

Each of the first contact sensor 200a and the second contact sensor 200b may be implemented by a contact pad, a ground portion, a resistor, a capacitor, etc., and may be implemented by any sensor capable of detecting that the apparatus for irradiating the skin with the light is in contact with the user's skin in addition to such an exemplary configuration.

The display unit 30 may be implemented by a flexible numeric display (FND) or the like, and performs various types of information output functions of displaying a menu of the apparatus for irradiating the skin with the light, displaying a light irradiation time, displaying battery residual capacity, displaying whether a blue LED operates, displaying whether a red LED operates, displaying various types of notification content, etc. This display unit 30 may be installed in a front surface case (or housing) of a main body of the apparatus for irradiating the skin with the light as illustrated in FIG. 2, and may be configured so that a display is viewed from the outside through a transparent or thin case material. In this manner, the display unit 30 is configured inside the transparent or thin case in consideration of the fact that the front surface case of the main body of the apparatus for irradiating the skin with the light is in contact with the user's skin.

In particular, the apparatus for irradiating the skin with the light according to the present invention comprises the light source unit 100 for irradiating the user's skin with medical light, the contact sensor 200 provided around the light source unit 100, and the controller 300 configured to control a light intensity of medical light to be radiated from the light source unit 100 according to whether the contact sensor 200 has detected the skin contact.

The controller 300 may cause the light source of the light source unit 100 to be selectively or sequentially driven. That is, only one of light sources of at least two colors may be driven according to a menu option or the light sources of the at least two colors may be driven sequentially.

For example, according to the menu option selected by the menu button 20, the controller 300 may cause only the blue LEDs to be driven, cause only the red LEDs to be driven, or cause the red LEDs to be driven after causing the blue LEDs to be first driven.

The controller 300 may cause the light source unit 200 to be driven according to a set time.

For example, the controller 300 may cause only the blue or red LEDs to be driven for five minutes or cause the red LEDs to be driven for 2 minutes and 30 seconds after causing the blue LEDs to be driven for 2 minutes and 30 seconds

The controller 300 may start light irradiation by driving the light source unit 100 according to an operation of the power supply button 10, and radiate medical light by controlling the light intensity of the above-described light to be increased from the moment at which the contact sensor 200 detects the skin contact when a menu option is selected by the menu button 20.

That is, when the contact sensor 200 is in contact with the user's skin, a contact signal is input to the above-described controller 300 and the controller 300 causes the medical light to be radiated by increasing the light intensity of the light source unit 100. As illustrated in FIG. 3a, when the power supply is turned on according to the operation of the power supply button (power supply ON), the controller 300 controls a pulse to be generated at predetermined cycle intervals during a standby mode before the skin contact is detected and maintains the pulse without an OFF section if the skin contact is detected. Thus, as illustrated in FIG. 3b, the light source unit 100 may maintain a light intensity (second light intensity or medical light intensity) sufficient for treatment by increasing the light intensity from the moment at which the skin contact is detected while a fixed light intensity (first light intensity or standby light intensity) of an extent at which it is not harmful to the user's eyes is maintained after the power supply is turned on. Through the above-described configuration, there is an advantageous effect in that safety may be improved by preventing the user's eyes from being exposed to excessively high-intensity medical light.

The controller 300 causes a light source to be operated in a corresponding menu option according to menu option selection through the menu button 20 before the skin contact is detected after the power supply is turned on during the standby mode. Through this, the user may recognize a light source to be operated in each menu option. For example, the controller 300 may cause only the blue light source of the light source unit 100 to be operated with a first light intensity when the user selects a first mode (or program mode) using the menu button 20, cause both of the blue and red light sources with the first light intensity when a second mode is selected, and cause only the red light source to be operated with the first light intensity when a third mode is selected. When the skin contact is achieved in the standby mode as described above, the controller 300 may cause the light intensity of the light source according to a corresponding menu option to be increased to a second light intensity.

Although not illustrated, the apparatus for irradiating the skin with the light according to the present invention may further include a temperature sensor. When a temperature detected by the temperature sensor exceeds a set temperature during medical light irradiation, the controller 300 may stop the driving of the light source unit 100. In addition, the apparatus for irradiating the skin with the light according to the present invention may further include a notification sound generation means such as a buzzer configured to generate a beep sound. Various types of notification sounds, for example, various notification sounds representing various notification content such as power supply ON/OFF notification, menu change notification, skin contact notification, skin contact release notification, error occurrence notification, and circuit interrupt notification due to an over-temperature or over-voltage, may be output through the notification sound generation means.

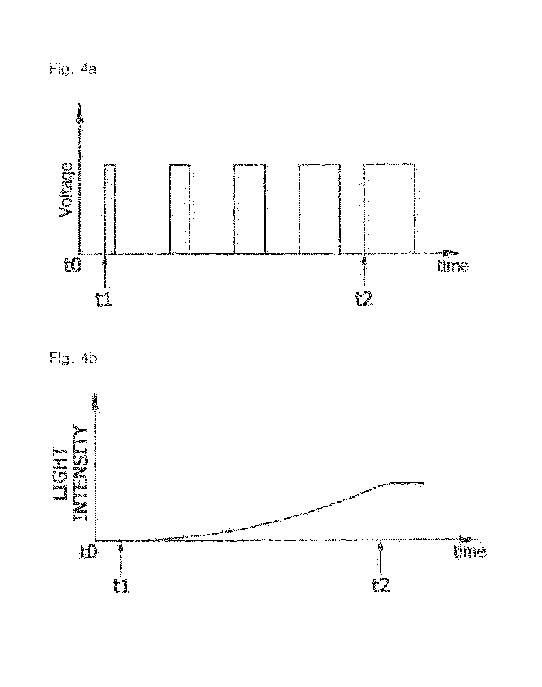

As a second embodiment, the controller 300 may start to radiate medical light after a first predetermined time t1 has elapsed from a time t0 at which the contact sensor 200 was detected the skin contact. In this case, the controller may control the light source unit 100 to gradually increase the light intensity of the radiated medical light and maintain the brightness of a fixed range after a second predetermined time t2. Referring to FIGS. 4a and 4b, the controller 300 controls the medical light to be radiated after the passage of the first predetermined time t1 when a skin contact signal of the user is input [t0] through the contact sensor 200 of the apparatus for irradiating the skin with the light, and controls the light intensity to be increased according to the passage of time and to be ultimately constant after the passage of the second predetermined time t2 [maintains the uniform brightness in a fixed range].

FIGS. 4a and 4b are graphs each illustrating a PWM method among light intensity control methods for the medical light according to the second embodiment of the present invention, wherein FIG. 4a is a graph illustrating the adjustment of a voltage pulse width according to time and FIG. 4b is a graph illustrating a light intensity variation amount according to time. The controller 300 may cause the light intensity of the medical light to be increased in a PWM method. As illustrated in FIG. 4a, the light intensity of the medical light to be radiated may be controlled to be gradually increased by increasing a duty ratio of a voltage pulse (a ratio between a high-level section and a low-level section of the pulse). That is, the controller 300 causes the light intensity of the medical light to be radiated to be gradually increased by gradually increasing a pulse width of a voltage output from the LED driver 100a from the time t1 which is a time at which a fixed time has elapsed from the time t0 at which the user's skin contact was detected to the time t2. The controller 300 causes the pulse width of the voltage output from the LED driver 100a to be constantly maintained at a predetermined magnitude after the time t2 has elapsed, thereby constantly maintaining the light intensity of the medical light to be radiated at a predetermined magnitude. Here, it is preferable that the light intensity of the medical light with a fixed predetermined magnitude to be radiated from the time t2, for example, be a magnitude of the light intensity to be actually used for acne treatment.

The controller 300 may cause the light intensity of the medical light to be increased through a PFM or an application voltage adjustment method in addition to the PWM method.

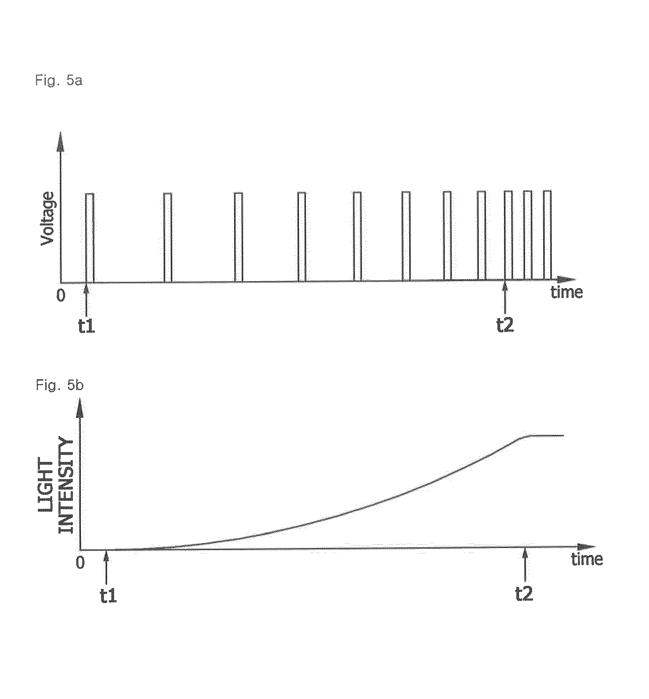

FIGS. 5a and 5b are graphs each illustrating a PFM method among the light intensity control methods for the medical light according to the second embodiment of the present invention, wherein FIG. 5a is a graph illustrating the adjustment of a voltage pulse frequency according to time and FIG. 5b is a graph illustrating a light intensity variation amount according to time. As illustrated in FIGS. 5a and 5b, the light intensity of the medical light to be radiated may be controlled to be gradually increased by increasing an iterative frequency (pulse cycle) of a voltage pulse. That is, the controller 300 causes the light intensity of the medical light to be radiated to be gradually increased by gradually increasing a pulse frequency of a voltage output from the LED driver 100a from the time t1 which is a time at which a fixed time has elapsed from the time t0 at which the user's skin contact was detected to the time t2. The controller 300 causes the pulse frequency of the voltage output from the LED driver 100a to be constantly maintained at a predetermined magnitude from the time t2, thereby constantly maintaining the light intensity of the medical light to be radiated at a predetermined magnitude. Here, it is preferable that the light intensity of the medical light with a fixed predetermined magnitude to be radiated from the time t2, for example, be a magnitude of the light intensity to be actually used for acne treatment.

Of course, in the present invention, the light intensity of the medical light to be radiated may be gradually increased and constantly maintained at a predetermined magnitude in a hybrid modulation method to which both the PWM and the PFM are applied.

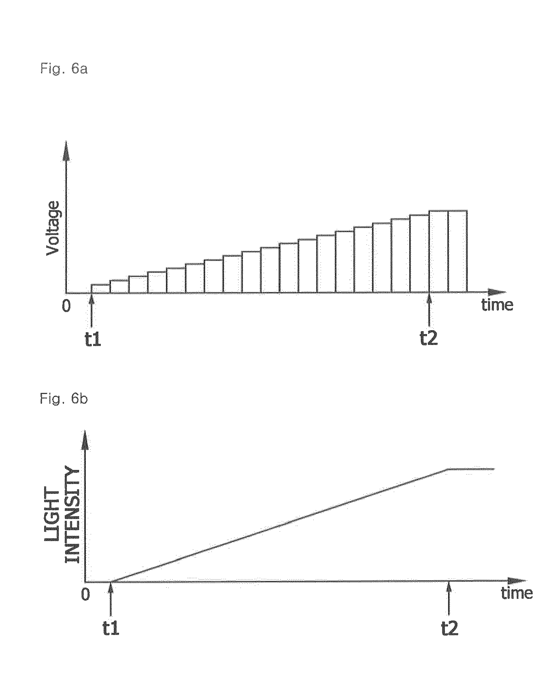

FIGS. 6a and 6b are graphs each illustrating an application voltage adjustment method among the light intensity control methods for the medical light according to the second embodiment of the present invention, wherein FIG. 6a is a graph illustrating the adjustment of an application voltage according to time and FIG. 6b is a graph illustrating a light intensity variation amount according to time. As illustrated in FIGS. 6a and 6b, the light intensity of the medical light to be radiated may be controlled to be increased by increasing a voltage to be applied to the light source unit 100. That is, the controller 300 causes the light intensity of the medical light to be radiated to be gradually increased by gradually increasing an application voltage from the time t1 which is a time at which a fixed time has elapsed from the time t0 at which the skin contact was detected to the time t2. The controller 300 causes the application voltage to be constantly maintained at a predetermined magnitude from the time t2, thereby constantly maintaining the light intensity of the medical light to be radiated at a predetermined magnitude. Here, it is preferable that the light intensity of the medical light with a fixed predetermined magnitude to be radiated from the time t2, for example, be a magnitude of the light intensity to be actually used for acne treatment.

It is obvious to those skilled in the art that all the PWM method, the PFM method, and the voltage application method as described above are applicable to embodiments of the present invention and all other light intensity control methods equivalent thereto are applicable to the embodiments of the present invention.

When a configuration is made so that the light intensity of the medical light is gradually increased, it is possible to prevent the user's skin from being suddenly exposed to a large amount of light and improve the safety of the apparatus for irradiating the skin with the light and the convenience of the user. Also, there is an advantage in that the apparatus for irradiating the skin with the light is safe because high-intensity light is not radiated to the user's eyes when the user separates the apparatus from the skin before the apparatus is completely closely in contact with the skin (for example, when the apparatus for irradiating the skin with the light is moved to an accurate portion to be treated due to wrong selection of a portion to be treated, when the apparatus is separated from the skin so as to receive a phone call or makes a response to a call from someone, or the like).

When the contact sensor 200 is separated from the user's skin while the medical light is radiated, the controller 300 may switch the mode to the standby mode and control a pulse to be generated at predetermined cycle intervals, thereby decreasing the light intensity to the first light intensity. In addition, the controller 300 may turn off the power supply when the skin contact is not detected within a predetermined time after the contact sensor 200 is separated from the user's skin, and may switch the mode to an operation mode when the skin contact is detected again within a predetermined time and maintain the pulse without an OFF section, thereby increasing the light intensity to the second light intensity again. For this, the controller 300 may check whether the contact sensor 200 is in contact with the skin at any time interval in a range from several milliseconds to several hundreds of milliseconds after the light irradiation starts, preferably, at intervals of 0.025 sec. This is because there may be a problem in that the user's eyes are exposed to excessively high-intensity medical light when the user momentarily separates the apparatus for irradiating the skin with the light if the reaction rate of the controller 300 to the detection signal of the contact sensor 200 is slow. Accordingly, it is preferable to configure the controller 300 in which the presence/absence of the skin contact of the contact sensor 200 is checked at intervals from several milliseconds to several hundreds of milliseconds so that the medical light irradiation may be stopped at the moment at which the user separates the apparatus for irradiating the skin with the light. Through this, it is possible to improve the safety of the apparatus for irradiating the skin with the light.

Alternatively, the controller 300 may cause the medical light irradiation to be stopped immediately when the contact sensor 200 is separated from the user's skin during the medical light irradiation.

On the other hand, the contact sensor is configured to surround a head portion of the conventional apparatus for irradiating the skin with the light. In this configuration, there is a problem in that the contact sensor detects the contact when the user's hand is in contact with the head portion and the user's eyes are directly exposed to light because the light is radiated from the light source unit.

In order to solve the above-described problem, a plurality of contact sensors 200 may be provided to oppose each other around the light source unit 100 in this embodiment. For example, as illustrated in FIG. 2, the first contact sensor 200a and the second contact sensor 200b are provided, and medical light may be radiated only when both the first contact sensor 200a and the second contact sensor 200b are in contact with the user's skin. This is to prevent the medical light from being radiated even when the user erroneously touches the contact sensor 200. Through this, there is an advantage in that safety may be improved.

In order to prevent all the plurality of contact sensors from being erroneously in contact with the user's hand, it is preferable to maximally separate the first contact sensor 200a from the second contact sensor 200b.

For this, in this embodiment, the first contact sensor 200a and the second contact sensor 200b may be provided to oppose each other in a length direction of the light source unit 100 or a width direction of the light source unit 100. Preferably, as illustrated in FIG. 2, the first contact sensor 200a and the second contact sensor 200b may be provided to oppose each other in the length direction of the light source unit 100. This is to prevent the contact sensor 200 from being in contact with the hand due to holding of both side surfaces of the apparatus for irradiating the skin with the light with the hand when the apparatus is in use.

On the other hand, as a third embodiment, the apparatus for irradiating the skin with the light may further include an optical detection unit (for example, a photo detector, a photo sensor, a light-receiving element, or the like) configured to detect external light provided on a specific portion (for example, a portion which is closely in contact with the user's skin such as a side portion of the contact sensor, a portion far away from the contact sensor, or a portion between the LEDs of the light source unit) of the front surface of the main body. In this embodiment, at least one contact sensor (one contact sensor, two contact sensors, or the like) may be configured.

In this embodiment, the skin contact is detected by the contact sensor and the irradiation of the medical light is controlled to start when the optical detection unit does not detect external light. That is, although the skin contact is accurately detected by the contact sensor, the medical light irradiation starts even when the contact sensor detects the contact with the user's hand. Because of this, it is more accurately detected whether the apparatus for irradiating the skin with the light is actually in contact with a facial surface of the user instead of the hand using the optical detection unit.

For example, because the optical detection unit is exposed to the external light when the apparatus for irradiating the skin with the light is not closely in contact with the user's skin, the medical light irradiation does not start even when the contact sensor detects the skin contact. When the apparatus for irradiating the skin with the light is closely in contact with the user's skin, the optical detection unit is shield from the external light and detects that there is no optical detection. Through this, the medical light irradiation starts by determining that the apparatus for irradiating the skin with the light is actually completely closely in contact with the facial surface of the user or the like.

On the other hand, as a fourth embodiment, the apparatus for irradiating the skin with the light may further include an optical detection unit (for example, a photo detector, a photo sensor, a light-receiving element, or the like) configured to detect external light provided on a specific portion (for example, a portion which is closely in contact with the user's skin such as a side portion of the contact sensor, a portion far away from the contact sensor, or a portion between the LEDs of the light source unit) of the front surface of the main body. In this embodiment, at least one contact sensor (one contact sensor, two contact sensors, or the like) may be configured.

In this embodiment, when the skin contact is not detected by the contact sensor and the external light is detected by the optical detection unit in a state in which the power supply is turned on or when the skin contact is detected by the contact sensor and the external light is detected by the optical detection unit, light irradiation of a predetermined magnitude (for example, weak light capable of providing notification of an LED operation) is performed from the light source unit.

That is, in this embodiment, the medical light irradiation is performed when the contact sensor detects the skin contact. In this case, because the irradiation starts after the apparatus for irradiating the skin with the light is closely in contact with the user's skin, the user does not know whether the LED of the light source unit operates with the eyes. For example, the user may desire to check whether the light source unit is well operated before the apparatus for irradiating the skin with the light is closely in contact with his/her skin.

Accordingly, the user externally exposes the apparatus for irradiating the skin with the light for use and therefore the optical detection unit is exposed to external light. In this embodiment, when the external light is detected by the optical detection unit, the light source unit radiates weak light. After the user checks whether the LED of the light source unit is well operated before close contact with the skin, the skin may be irradiated with medical light by causing the apparatus to be closely in contact with the skin.

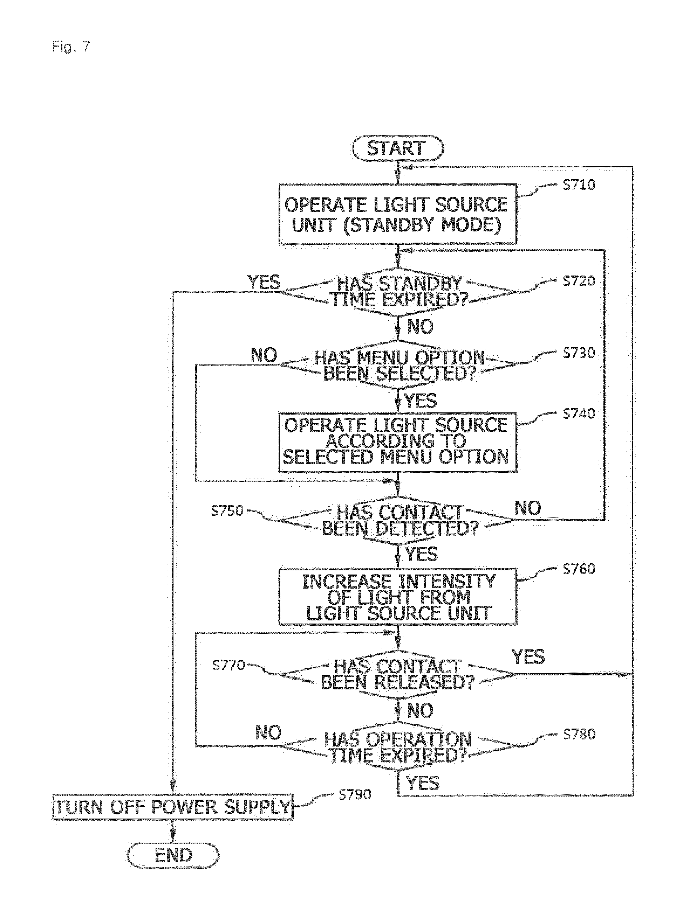

FIG. 7 is a flowchart illustrating a method of operating the apparatus for irradiating the skin with the light according to the first embodiment of the present invention.

Referring to FIG. 7, when the power supply button 10 is pressed, the controller 300 enters the standby mode in step S710, and causes the light source unit 100 to be driven with the first light intensity. At this time, a basic menu, for example, may cause the light source unit 100 to be driven in the first mode (or program mode). The controller 300 determines whether a standby time has expired in step S720, and stops the driving of the light source unit 100 by turning off the power supply in step S790 when the standby time has expired. Before the standby time expired, the controller 300 determines whether a menu option has been selected in step S730. When the menu option has been selected, the controller 300 causes the light source unit 100 to be driven with the first light intensity in the mode according to the selected menu option in step S740. As described above, according to the menu option, one of light sources of at least two colors may be driven or the light sources of the at least two colors may be driven sequentially. For example, three programs may be basically input to the apparatus for irradiating the skin with the light according to the present invention. Accordingly, the apparatus are operable in three modes (or program modes). A first program P1 may be designed so that blue light (with which skin sterilization or acne treatment is possible) is radiated for 5 minutes, a second program P2 may be designed so that red light (which is helpful for skin reproduction) is radiated for 2 minutes and 30 seconds after the blue light is radiated for 2 minutes and 30 seconds, and a third program P3 may be designed so that the red light is radiated for 5 minutes. That is, every time the menu button 20 is pressed once, the programs P1, P2, and P3 may be changed and this program change may be displayed on the display unit 30.

On the other hand, when the determination result of step S730 represents that the menu option has not been selected or after step S740 is performed, the controller 300 determines whether the contact has been detected in step S750. When the contact has not been detected, the process returns to step S720 and subsequent processes are performed. When the determination result of step S750 represents that the contact has been detected, the controller 300 causes the light source unit 100 to be driven by increasing the light intensity of the light source unit 100 to the second light intensity in step S760. At this time, as described above, the controller 300 checks the presence/absence of skin contact of the contact sensor 200 at intervals of several milliseconds or several hundreds of milliseconds after the initiation of light irradiation. When the determination result of step S770 represents that the skin contact has been released, the process returns to the standby mode in step S710 and subsequent processes are performed. When the light irradiation continues without the contact release, the controller 300 determines whether an operation time has expired in step S780. When the operation time has expired, the process returns to the standby mode of step S710 and subsequent processes are performed. Until the operation expires, the controller 300 iterates the check of presence/absence of the skin contact of the contact sensor 200 and the operation time expiration process (S770 and S780).

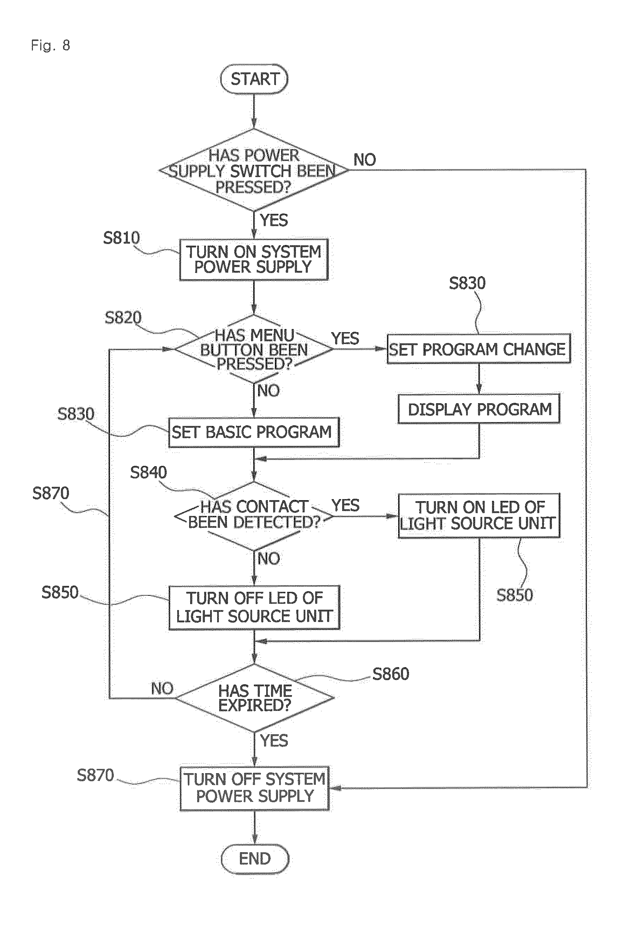

FIG. 8 is a flowchart illustrating a method of operating the apparatus for irradiating the skin with the light according to the second embodiment of the present invention.

The present invention provides the method of operating the apparatus for irradiating the skin with the light, the method including step S810 of operating the apparatus by pressing the power supply button 10, step S820 of determining whether the menu button 20 has been pressed, step S830 of changing a program to set the changed program when pressing of the menu button 20 is detected and setting a basic program when the pressing is not detected, step S840 of determining whether the plurality of contact sensors 200 have been all in contact with the user's skin, step S850 of radiating medical light from the light source unit 100 when the contact sensor 200 has detected the contact and stopping irradiation of the medical light when the contact has not been detected, step S860 of determining whether a set time has expired, and step S870 of turning off the power supply after the irradiation of the medical light is stopped when the setting time has expired and continuing the irradiation of the medical light when the preset time has not expired.

As illustrated in FIG. 8, the apparatus for irradiating the skin with the light is operated (step S810) when the power supply button 10 is pressed. Thereafter, it is determined whether the menu button 20 has been pressed (step S820).

As described above, every time the menu button 20 is pressed once, the programs P1, P2, and P3 are changeable (step S830). This program change is displayed on the display unit 30.

When the menu button 20 has not been pressed, the basic program (P1) is set (step S830). Thereafter, the process proceeds to the next step (step S840).

When the program is set, the process proceeds to the step (step S840) of determining whether all the contact sensors 200 have been in contact with the user's skin. When the contact has been detected in the contact sensors 200, the light source unit 100 starts to radiate light corresponding to the set program (step S850).

When the contact has not been detected in the contact sensors 200, the irradiation of the medical light is stopped (step S850).

When the irradiation of medical light starts, the process proceeds to the step (step S860) of determining whether the set time has expired. When the set time has not expired, the irradiation of the medical light continues (step S870). When the set time has expired, the operation of the apparatus for irradiating the skin with the light is stopped (step S870) after the irradiation of the medical light has been stopped.

The power supply may be turned on due to wrong pressing of a button when the conventional portable apparatus for irradiating skin with light is carried. Because of this, power of an embedded power supply unit may be unnecessarily consumed, the apparatus may not be used at a desired time due to the power consumption, an erroneous operation of the contact sensor may be caused, and a life of a treatment device may be shortened.

In order to solve the above-described problems, the apparatus for irradiating the skin with the light according to the present invention may have a lock function. The lock function may be set through step S880a of simultaneously pressing the power supply button 10 and the menu button 20 in a state in which the power supply of the apparatus is turned off, step S880b of performing the lock function in the controller 300 when a predetermined time ta has elapsed after the power supply button 10 and the menu button 20 were pressed simultaneously, and step S880c of displaying the lock function on the display unit 300 when the power supply button 10 or the menu button 20 are pressed after the lock function is performed and interrupting power.

After the lock function is performed, the lock function should be released when the apparatus for irradiating the skin with the light is reused. An unlock function includes step S890a of simultaneously pressing the power supply button 10 and the menu button 20 after the power is interrupted and step S890b of performing the unlock function in the controller 300 when a predetermined time tb has elapsed after the power supply button 10 and the menu button 20 were pressed simultaneously.

The time ta and the time tb may be the same as or different from each other, and may be preferably adjusted.

Although the present invention has been described above in details with reference to preferred embodiments, the scope of the present invention is not limited to a specific embodiment but may be construed only in view of the appended claims. It is to be understood by those skilled in the art that various changes and modifications may be made without departing from the scope of the present invention.

## Claims

1. An apparatus for irradiating skin with light, comprising:
an input unit for a user input;
a light source unit including light sources of at least two colors and configured to irradiate the skin of a user with medical light;
a contact sensor provided at periphery of the light source unit; and
a controller configured to control an intensity of the light radiated from the light source unit according to whether skin contact is detected by the contact sensor,
wherein the controller controls the intensity of the light from the light source unit in at least two stages before and after the skin contact is detected.

2. The apparatus for irradiating the skin with the light according to claim 1,
wherein the controller drives the light source unit with a first light intensity before the skin contact is detected by the contact sensor after a voltage is applied, and
wherein the controller drives the light source unit with a second light intensity when the skin contact is detected.

3. The apparatus for irradiating the skin with the light according to claim 2, wherein the controller decreases the intensity of the light from the light source unit to the first light intensity when the skin contact is released while the medical light is radiated.

4. The apparatus for irradiating the skin with the light according to claim 3,
wherein the controller drives the light source unit for a set driving time if the skin contact is not released while the medical light is radiated, and
wherein the controller decreases the intensity of the light from the light source unit to the first light intensity when the driving time has expired.

5. The apparatus for irradiating the skin with the light according to claim 2, wherein the controller causes the light intensity to be gradually increased until the light source unit reaches the first or second light intensity.

6. The apparatus for irradiating the skin with the light according to claim 1, wherein the controller controls at least one of the light sources of the at least two colors to be selectively or sequentially driven.

7. The apparatus for irradiating the skin with the light according to claim 6, wherein the light sources of the at least two colors include a blue light source and a red light source.

8. The apparatus for irradiating the skin with the light according to claim 7, wherein the controller drives only one of the blue light source and the red light source according to the input to the input unit.

9. The apparatus for irradiating the skin with the light according to claim 7, wherein the controller first drives one of the blue light source and the red light source according to the input to the input unit and subsequently drives the other.

10. The apparatus for irradiating the skin with the light according to claim 1, further comprising at least one of:
a display unit configured to display various types of device states;
a temperature sensor configured to detect a temperature so as to prevent an over-temperature of the light source unit; and
a notification sound generation means configured to output a notification sound.

11. The apparatus for irradiating the skin with the light according to claim 1, wherein a plurality of contact sensors are provided to oppose each other around the light source unit.

12. The apparatus for irradiating the skin with the light according to claim 11, wherein the controller starts to radiate the medical light only when the plurality of contact sensors all detect the skin contact.

13. The apparatus for irradiating the skin with the light according to claim 1, further comprising:
an optical detection unit configured to detect external light.

14. The apparatus for irradiating the skin with the light according to claim 13, wherein the controller starts to radiate the medical light when the external light is not detected by the optical detection unit.
